# EUROPÄISCHE PATENTANMELDUNG

(11) **EP 2 468 354 A2**
(43) Veröffentlichungstag der Anmeldung: **27.06.2012**
(21) Anmeldenummer: 11193180.4
(22) Anmeldetag: 13.12.2011
(51) Int. Cl.: A61N 1/05, A61N 1/36, A61N 1/08, A61N 1/37, A61N 1/39

(54) **Implantierbares Gerät**

(30) Priorität: 21.12.2010 US 201061425255 P
(71) Anmelder: BIOTRONIK SE & Co. KG, 12359 Berlin (DE)
(72) Erfinder: Weiss, Ingo, 12435 Berlin (DE); Knorr, Stefan, 10119 Berlin (DE); Diebold, Michael, 12169 Berlin (DE); Fandrey, Stephan, 8910 Affoltern am Albis (CH)
(74) Vertreter: Lindner-Vogt, Karin L.

(57) **Zusammenfassung**

Temporär oder permanent implantierbares medizinisches Gerät mit mindestens einer langgestreckten elektrischen Funktionsleiter,
- der distal mit einem Elektrodenpol zur Abgabe von Therapiesignalen oder zur Erfassung von Diagnosesignalen verbunden ist,
- der in einem Längsabschnitt eine Strombegrenzungsvorrichtung aufweist, welche eine Parallelschaltung eines ersten und eines zweiten Strombegrenzers aufweist, von denen jeder ausgebildet ist, ein Überschreiten eines vorgegebenen Maximalwertes einer elektrischen Stromstärke im elektrischen Leiter zu verhindern,
- wobei der zweite Strombegrenzer zusätzlich ausgebildet ist, bei Anliegen einer elektrischen Betriebsspannung und gleichzeitigem Unterschreiten eines vorgegebenen, zum zweckgemäßen Betrieb des Gerätes erforderlichen Minimalwerts der elektrischen Stromstärke in einem ersten, nur den ersten der Strombegrenzer enthaltenden Stromzweig der Parallelschaltung, in einem zum ersten Stromzweig parallelen zweiten Stromzweig der Parallelschaltung, der nur den zweiten der Strombegrenzer enthält, eine elektrische Stromstärke zumindest des Minimalwerts zu gestatten.

## Beschreibung

Die Erfindung betrifft ein permanent oder temporär implantierbares medizinisches Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse, beispielsweise an das Gewebe (Myokard) des Herzens oder zum Abfühlen elektrischer Felder, um im Rahmen des sogenannten Sensings eine Aktivität, beispielsweise eine Herzaktivität abfühlen zu können.

Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen.

Die Elektrodenpole sind über einen oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalem Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleiter bezeichnet.

Solche Funktionsleiter sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Aus der EP 2 067 501 A2 ist die Verwendung einer Diode zur Strombegrenzung in einer Elektrodenleitung bekannt. Diese Lösung hat den Vorteil, dass sie von der Frequenz der einwirkenden Störfelder und der dadurch induzierten Ströme weitgehend unabhängig ist. Die relativ hohen Spannungen, denen eine in Sperrrichtung geschaltete Diode insbesondere bei starken Leistungen einwirkender magnetischer Wechselfelder in einem Magnetresonanz-Gerät ausgesetzt ist, können jedoch zur Zerstörung der Diode führen. Dadurch wird die Diode nichtleitend. Dies führt bei einem Herzschrittmacher oder Defibrillator zum Verlust der therapeutischen oder diagnostischen Wirkung eines Elektrodenpols, der durch eine zerstörte und daher nichtleitende Diode vom Steuergerät abgekoppelt ist.

Die genannte bekannte Lösung ist auch empfindlich gegen mechanische Beanspruchung. Denn Halbleiter sind an sich mechanisch sehr fragil und müssen über eine ebenfalls mechanisch anfällige Bondtechnik an metallische Kontakte angeschlossen werden.

Der Erfindung liegt die Aufgabe zugrunde, ein medizinisches Gerät zu schaffen, welches das zuvor beschriebene Problem lindert oder löst.

Erfindungsgemäß wird diese Aufgabe gelöst durch ein Temporär oder permanent implantierbares medizinisches Gerät mit mindestens einem langgestreckten elektrischen Funktionsleiter,
- der distal mit einem Elektrodenpol zur Abgabe von Therapiesignalen oder zur Erfassung von Diagnosesignalen verbunden ist, und
- der in einem Längsabschnitt eine Strombegrenzungsvorrichtung aufweist, welche eine Parallelschaltung eines ersten und eines zweiten Strombegrenzers aufweist, von denen jeder ausgebildet ist, ein Überschreiten eines vorgegebenen Maximalwertes einer elektrischen Stromstärke im elektrischen Leiter zu verhindern.

Der zweite Strombegrenzer ist erfindungsgemäß zusätzlich ausgebildet, bei Anliegen einer elektrischen Betriebsspannung und gleichzeitigem Unterschreiten eines vorgegebenen Minimalwerts der elektrischen Stromstärke in einem ersten, nur den ersten der Strombegrenzer enthaltenden Stromzweig der Parallelschaltung einen vorgegebenen, zum zweckgemäßen Betrieb des Gerätes erforderlichen Minimalwert der elektrischen Stromstärke in einem zum ersten Stromzweig parallelen zweiten Stromzweig der Parallelschaltung zu gestatten, der nur den zweiten der Strombegrenzer enthält.

Durch die erfindungsgemäße Lösung wird der im elektrischen Funktionsleiter fließende Stromfluss zum Elektrodenpol auch in dem Falle ermöglicht, dass der erste Strombegrenzer seine elektrische Leitfähigkeit aufgrund eines Fehlers, so dass ein über den Elektrodenpol in das Gewebe fließender Strom für die gewünschte therapeutische oder diagnostische Wirkung ausreicht. Andererseits wird der Stromfluss durch die Wirkung des zweiten Strombegrenzers jedoch zugleich insoweit eingeschränkt, dass eine potentielle Erwärmung durch unerwünschte Ströme, wie sie beispielsweise aus Störfeldern induziert werden können, weitestgehend vermieden wird.

Die mechanisch meist fragile Implementierung eines Strombegrenzers wird durch die erfindungsgemäße vorgesehene Parallelschaltung zweier Strombegrenzer um einen vom erwähnten zweiten Stromzweig gebildeten Sicherheitspfad ergänzt. Dieser kann in bevorzugten Beispielen mechanisch robust ausgebildet sein, wie weiter unten näher beschrieben wird.

Nachfolgend werden Ausführungsbeispiele des erfindungsgemäßen medizinischen Geräts beschrieben. Die zusätzlichen Merkmale der Ausführungsbeispiele können miteinander kombiniert werden, um weitere Ausführungsbeispiele zu bilden, soweit sie nicht ausdrücklich als Alternativen zueinander beschrieben werden.

Der erste Strombegrenzer ist bevorzugt ausgebildet, bei Anliegen einer Sperrspannung definierter Polarität und Amplitude am ersten Strombegrenzer den ersten Stromzweig zu sperren.

Bei unterschiedlichen alternativen Ausführungsbeispielen ist eine elektrische Leitfähigkeit des ersten Strombegrenzers abhängig entweder von einer Frequenz eines Wechselfeldes, dem der erste Stromzweig ausgesetzt ist, oder von einer über dem ersten Stromzweig abfallenden elektrischen Spannung, oder von einem Betrag oder einer Richtung eines elektrischen oder magnetischen Feldes, dem der erste Stromzweig ausgesetzt ist, oder von einer Temperatur am ersten Stromzweig, oder von einer Kombination mindestens zweier der genannten Größen.

Bevorzugte Ausführungsbeispiele medizinischer Geräte der vorliegenden Erfindung enthalten als ersten Strombegrenzer mindestens eine Diode oder mindestens einen Transistor, oder einen Schaltkreis mit mindestens einer Diode und mindestens einem Transistor. Als Transistor eignen sich in alternativen Ausführungsbeispielen ein Sperrschicht-Feldeffekttransistor, kurz JFET, ein Metall-Halbleiter-Feldeffekttransistor, kurz MESFET, ein Feldeffekt-Transistor mit hoher Elektronenbeweglichkeit, kurz HEMT, insbesondere ein pseudomorpher HEMT, kurz pHEMT, oder ein Metall-Oxid-Halbleiter-Feldeffekttransistor, kurz MOSFET. In einer Variante ist der erste Strombegrenzer von zwei in Reihe geschalteten Transistoren gebildet, deren Gate-Elektroden direkt miteinander verbunden sind.

Der erste Strombegrenzer ist vorzugsweise als monolithisch integriertes Halbleiterbauelementausgebildet. Als solches kann er in Form eines Hohlzylinders mit Kontaktflächen an seinen ringförmigen Längsenden ausgeführt sein. Der Hohlzylinder umgibt den Funktionsleiter und, bei Vorliegen eines Geräts mit Abschnitten unterschiedlicher Leitfähigkeit, den Längsabschnitt geringerer elektrischer Leitfähigkeit umgibt.

Bei einem Ausführungsbeispiel bei dem weist Funktionsleiter an einem proximalen Ende seiner Längserstreckung eine Anschlussvorrichtung zum Anschluss an eine Steuervorrichtung des Geräts auf. An einem entgegengesetzten, distalen Ende seiner Längserstreckung weist er mindestens einen zur Abgabe elektrischer Stimulationsimpulse an stimulierbares biologisches Gewebe geeigneten Elektrodenpol auf, wobei die Strombegrenzungsvorrichtung nahe dem distalen Ende des Leiters angeordnet ist.
Vorzugsweise ist die Strombegrenzungsvorrichtung maximal 5 cm der Längserstreckung des Leiters vom Elektrodenpol entfernt.

In einer Ausführungsform ist der Funktionsleiter mit Überbrückungskontakten verbunden, die in Längsrichtung beiderseits der Strombegrenzungsvorrichtung angeordnet sind und die ausgebildet sind, durch Verbinden mit einer Überbrückungsvorrichtung direkt miteinander verbunden zu werden.

Ein Ausführungsbeispiel weist zusätzlich eine Steuervorrichtung auf, die mit dem Funktionsleiter verbunden ist und die umfasst:
- eine Mess-Einheit, die ausgebildet ist, mit einem Messfühler einen Messwert einer von der elektrischen Stromstärke im Leiter verschiedenen Messgröße zu ermitteln, die geeignet ist, auf das Auftreten einer den Maximalwert überschreitenden elektrischen Stromstärke im Leiter hinzuweisen,
- eine Auswerte-Einheit, die ausgebildet ist, anhand des Messwerts das Vorliegen einer den Maximalwert überschreitenden elektrischen Stromstärke im Funktionsleiter zu erfassen und gegebenenfalls ein Steuersignal zu erzeugen,
- eine Schalt-Einheit, die mit der Steuervorrichtung und dem ersten Strombegrenzer verbunden ist und die ausgebildet ist, bei Anliegen des Steuersignals eine zum Sperren des durch den ersten Strombegrenzer führenden ersten Stromzweiges geeignete Sperrspannung zu erzeugen und an den Funktionsleiter anzulegen.

Ein solches Medizinisches Gerät hat in seiner Steuervorrichtung vorzugsweise eine Therapie-Einheit, die mit der Auswerte-Einheit verbunden und ausgebildet ist,
- elektrische Stimulationsimpulse mit einem vordefinierten zeitlichen Verlauf ihrer Amplitude zu erzeugen, die einen vorgegebenen Sollwert der elektrischen Stromstärke im Funktionsleiter bewirken, wobei der Sollwert größer ist als der Minimalwert und kleiner als der Maximalwert,
- die Stimulationsimpulse über den Funktionsleiter nach extern abzugeben, und
- bei Anliegen des von der Auswerte-Einheit ausgegebenen Steuersignals den zeitlichen Verlauf der Amplitude der Stimulationsimpulse im Funktionsleiter zu ändern.

Damit auch über diesen die Therapie erfolgreich ist, sieht das medizinische Gerät vorzugsweise einen Rückkopplungspfad vor, über den die Therapieparameter entsprechend angepasst werden, um die Patientensicherheit in jedem Fall zu gewähren. Die Mess-Einheit ist in einem Ausführungsbeispiel zusätzlich ausgebildet, einen aktuellen Wert einer Messgröße zu erfassen, die geeignet ist, eine Wirksamkeit oder eine Unwirksamkeit an das zu stimulierende Gewebe abgegebener Stimulationsimpulse anzuzeigen,
- die Auswerte-Einheit ausgebildet ist, anhand des aktuellen Werts und vordefinierter Kriterien die Wirksamkeit oder Unwirksamkeit der abgegebenen Stimulationsimpulse zu ermitteln, und
- die Therapie-Einheit ausgebildet ist, bei ermittelter Unwirksamkeit zeitlichen Verlauf der Amplitude der Stimulationsimpulse im Funktionsleiter zu ändern.

Der Messfühler ist vorzugsweise am Funktionsleiter angeordnet und über den zweiten Signalpfad mit der Auswerte-Einheit verbunden.

Zusätzlich kann im Medizinischen Gerät eine mit der Auswerte-Einheit verbundene Kommunikations-Einheit zur Kommunikation des Geräts mit einem externen Überwachungsgerät über einen Kommunikationskanal vorgesehen sein, wobei die Kommunikations-Einheit ausgebildet ist, bei Vorliegen des Steuersignals dem Überwachungsgerät auf dem Kommunikationskanal das Vorliegen einer den Maximalwert überschreitenden elektrischen Stromstärke im Funktionsleiter zu signalisieren.

Nachfolgend werden weitere Ausführungsbeispiele anhand der Figuren erläutert. Es zeigen:
- Fig. 1: ein Ausführungsbeispiel eines erfindungsgemäßen medizinischen Geräts in Form eines Herzschrittmachers;
- Fig. 2: eine vereinfachte Schaltskizze eines Ausführungsbeispiels einer Elektrodenleitung;
- Fig. 3: eine Strom-Spannungs-Kennlinie eines zur Verwendung im Beispiel der Fig. 2 geeigneten Strombegrenzers im Vergleich mit einer Ohm'schen Kennlinie;
- Fig. 4: eine vereinfachte Schaltskizze einer Elektrodenleitung nach einem weiteren Ausführungsbeispiel;
- Fig. 5A und 5B: eine schematische Darstellung einer Elektrodenleitung in einer seitlichen Ansicht und in einer Querschnittsansicht zur Erläuterung eines weiteren Ausführungsbeispiels;
- Fig. 6A und 6B: eine Schaltskizze und eine schematische Querschnittsansicht eines Strombegrenzers nach einem Ausführungsbeispiel;
- Fig. 7A und 7B: eine Schaltskizze und eine schematische Querschnittsansicht eines alternativen Strombegrenzers;
- Fig. 8: eine schematische dreidimensionale Darstellung eines Strombegrenzers;
- Fig. 9A: schematische dreidimensionale Darstellung einer alternativen Ausführungsform eines Strombegrenzers; und
- Fig. 9B: eine Querschnittsansicht eines weiteren Ausführungsbeispiels eines Strombegrenzers nach Fig. 8 oder 9a.

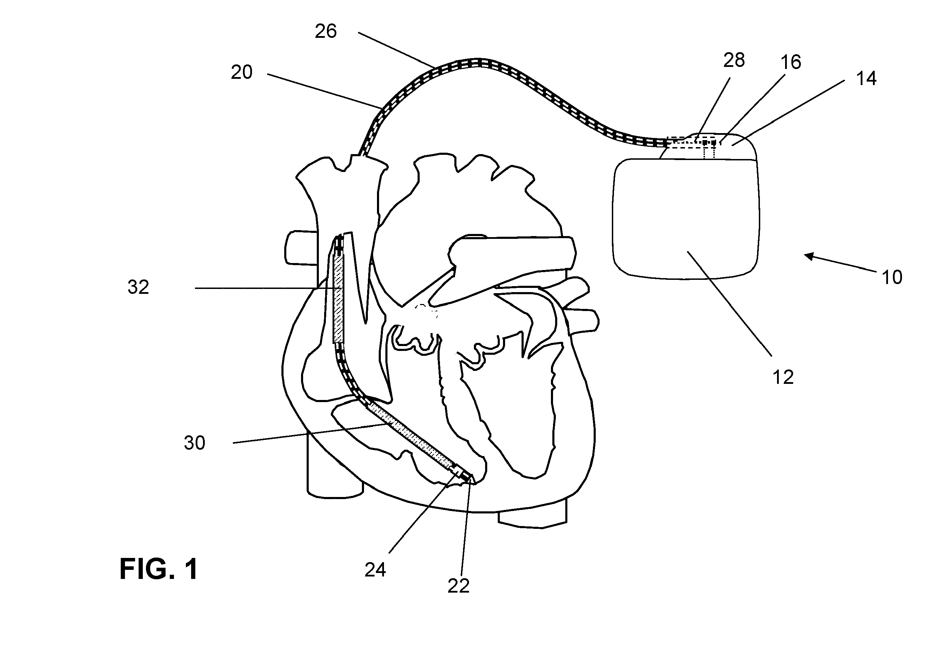

Fig. 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert wird, können die elektrischen Leiter 26 in der Elektrodenleitung 20 in unterschiedlichen Längsabschnitten als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

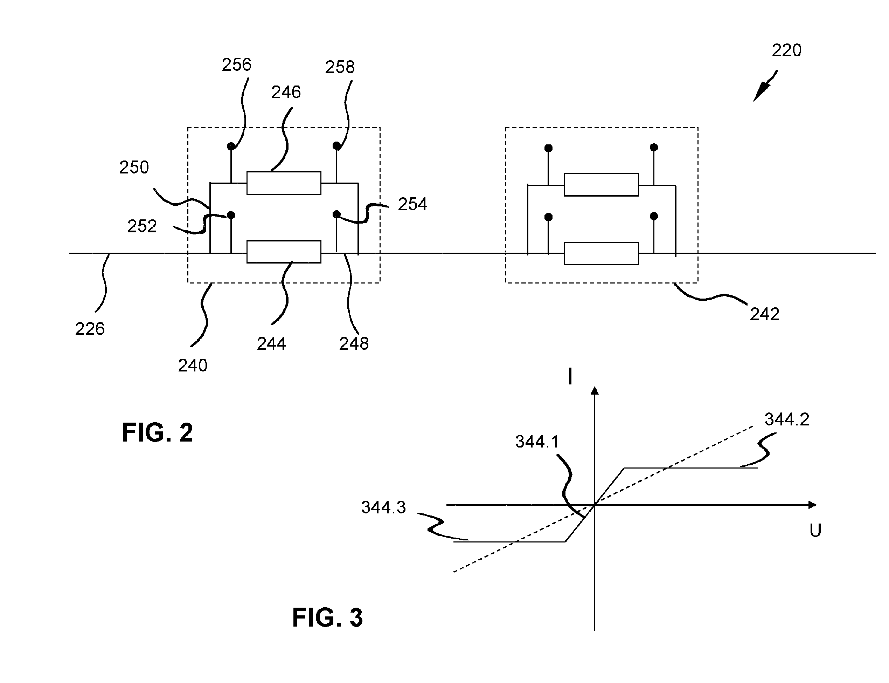

Fig. 2 ist eine vereinfachte Schaltskizze eines Ausführungsbeispiels einer Elektrodenleitung. Parallel wird auf Fig. 3 Bezug genommen, die eine Kennlinie des Strombegrenzers zeigt.

Die Elektrodenleitung 220 des vorliegenden Ausführungsbeispiels enthält einen (oder mehrere) Funktionsleiter 226. Der Funktionsleiter 226 dient zur Übertragung von therapeutischen und diagnostischen Signalen zwischen einem der distal angeordneten Elektrodenpole, wie sie in Fig. 1 mit den Bezugszeichen 30 und 32 gekennzeichnet sind, und dem Steckkontakt 28 am proximalen Ende der Elektrodenleitung 220. Dargestellt ist ein Längsabschnitt des Funktionsleiters 226, in welchem zwei Strombegrenzungsvorrichtungen 240 und 242 angeordnet sind. Die beiden Strombegrenzungsvorrichtungen 240 und 242 sind in Form einer Serienschaltung über den Funktionsleiter 226 miteinander verbunden. Der interne Aufbau der Strombegrenzungsvorrichtungen 240 und 242 ist identisch. Daher wird nachfolgend der Knappheit halber nur der Aufbau der Strombegrenzungsvorrichtung 240 näher beschrieben. Es sei erwähnt, dass die Anzahl der Strombegrenzungsvorrichtungen nach Bedarf variiert werden kann. Es kann abweichend von dem in Fig. 2 dargestellten Beispiel auch nur eine Strombegrenzungsvorrichtung vorgesehen werden. Alternativ ist es je nach Anwendungsfall auch möglich, mehr als zwei Strombegrenzungsvorrichtungen vorzusehen.

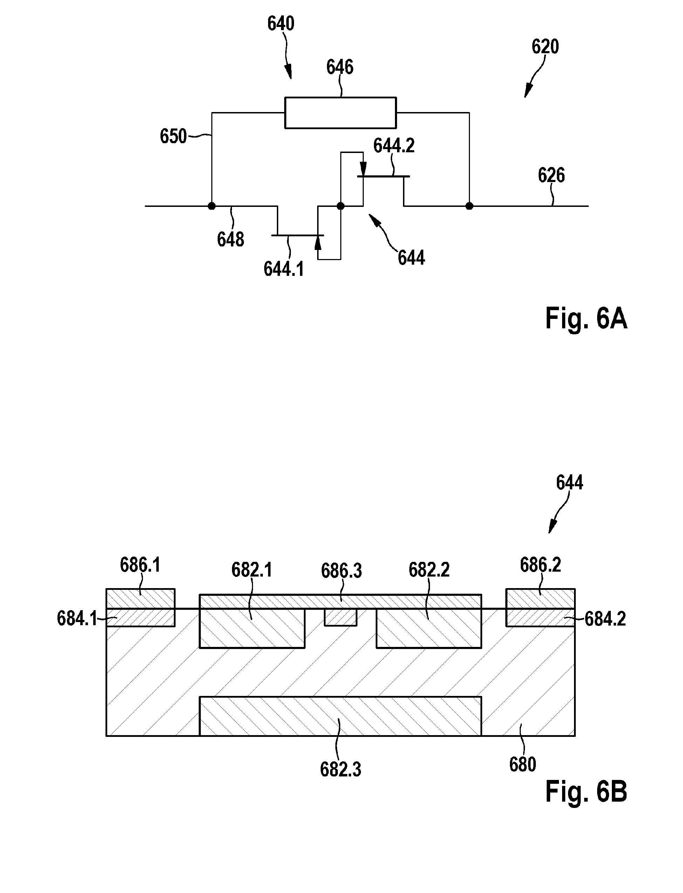

Die Strombegrenzungsvorrichtung 240 enthält eine Parallelschaltung eines ersten Strombegrenzers 244 und eines zweiten Strombegrenzers 246. Beide Strombegrenzer 244 und 246 dienen grundsätzlich dazu, ein Überschreiten eines vorgegebenen Maximalwerts einer elektrischen Stromstärke im Funktionsleiter zu verhindern. Auf konkrete Realisierungsformen des ersten und zweiten Strombegrenzers 244 und 246 wird für die Zwecke des vorliegenden Ausführungsbeispiels nicht eingegangen. Hierzu dienen die weiter unten vorgestellten Ausführungsformen der Fig. 6 bis 9. Aufgrund dieser Parallelschaltung des ersten und des zweiten Strombegrenzers sind zwei Stromzweige 248 und 250 vorhanden. Der erste Stromzweig 248 führt zwischen dem proximalen und dem distalen Ende des Funktionsleiters 226 durch den ersten Strombegrenzer 244. Der zweite Stromzweig 250 führt durch den zweiten Strombegrenzer 246. Beide Stromzweige 248 und 250 können jeweils überbrückt werden. Hierzu sind im ersten Stromzweig zugängliche Überbrückungskontakte 252 und 254 vorgesehen, und im zweiten Stromzweig 250 sind Überbrückungskontakte 256 und 258 zugänglich. Nachfolgend wird die Funktionsweise der Strombegrenzungsvorrichtung 240 näher erläutert. Die Fähigkeit des ersten Strombegrenzers 244, Strom zu leiten, hängt allgemein gesprochen von einem Parameter ab. Beispielsweise wird im Falle einer Induktion von elektrischem Strom in einem magnetischen Wechselfeld, wie es beispielsweise ein Magnetresonanztomograph darstellt, die zeitliche Änderung eines magnetischen Flusses, der einen den Funktionsleiter enthaltenden Stromkreis durchdringt, eine Induktionsspannung hervorrufen, welche den Induktionsstrom im Funktionsleiter bewirkt. Die Änderungsgeschwindigkeit, d. h. die zeitliche Ableitung des magnetischen Flusses, bestimmt die Höhe der Induktionsspannung und damit über die bekannten physikalischen Zusammenhänge auch die Stärke des induzierten Stroms. Bei einem magnetischen Wechselfeld einer für die Magnetresonanztomographie typischen hohen Frequenz sorgt der Effektivwert des induzierten Stromes für eine Erwärmung des Funktionsleiters, die insbesondere im Bereich der Elektrodenpole (in Fig. 2 nicht dargestellt) schädlich sein kann. Das Hochfrequenz-Feld eines Magnetresonanztomographen ist typischerweise gepulst mit einem Tastverhältnis von meist weit unter 10 %, weist aber recht hohe Spitzenwerte auf.

Die dadurch induzierten Stromspitzen werden mit dem Strombegrenzer 244, welcher typischerweise eine in Fig. 3 dargestellte Kennlinie 344 aufweist, weggeschnitten werden. Die Kennlinie 344 hat einen linearen Abschnitt 344.1, in welchem der Betrag der Stromstärke sich in etwa proportional zum Betrag der Spannung verhält. Daran schließen zu höheren positiven wie negativen Spannungswerten hin Sättigungsbereiche an, in denen der Strom bei weiter steigendem Betrag der Spannung im Wesentlichen konstant ist. Zum Vergleich ist in Fig. 3 mit einer gestrichelten Linie eine Ohm'sche Kennlinie dargestellt, die über den gesamten dargestellten Spannungsbereich eine lineare Abhängigkeit des Stroms zeigt.

Der im normalen Betriebszustand erforderliche Therapiestrom sollte im linearen Bereich 344.1 der Kennlinie des Strombegrenzers 244 liegen, sich also stets unterhalb der Schwelle der Strombegrenzung bewegen.

Der zweite Strombegrenzer 246 im zweiten Stromzweig 250 der Strombegrenzungsvorrichtung 240 ist von seiner grundsätzlichen Wirkungsweise her ähnlich ausgelegt wie der erste Strombegrenzer 244. Der zweite Strombegrenzer wird jedoch so ausgelegt, dass er in dem Fall, dass der erste Strombegrenzer 244 ausfällt, indem er seine Leitfähigkeit für elektrischen Strom einbüßt, in seiner Funktion ersetzen kann. Diese Situation ist typischerweise dadurch gekennzeichnet, dass im ersten Stromzweig der Strombegrenzungsvorrichtung bei Anlegen einer Betriebsspannung ein vorgegebener Minimalwert der elektrischen Stromstärke unterschritten wird. Der vorgegebene Minimalwert ist dadurch bestimmt, dass beispielsweise für therapeutische Zwecke eine ausreichend hohe Stromstärke fließen muss, um die gewünschte therapeutische Wirkung zu erzielen. Allgemein muss die im Funktionsleiter erzielbare Stromstärke zumindest den Minimalwert erreichen, um zweckgemäß betrieben werden zu können. Der zweite Strombegrenzer 246 stellt sicher, dass bei einem solchen Ausfall des ersten Strombegrenzers im Funktionsleiter 226 trotzdem ein Strom fließen kann, der den vorgegebenen Minimalwert zumindest erreicht.

Aufgrund der Parallelschaltung der beiden Strombegrenzer 244 und 246 muss im Vergleich mit einer Strombegrenzungsvorrichtung, die nur einen einzigen Strombegrenzer enthält, der Schwellwert der Stromstärke, ab welchem die Strom begrenzende Wirkung auftritt, für beide Strombegrenzer 244 und 246 geringer ausgelegt werden. Fällt der Strombegrenzer 244 aus, so wird der gesamte Strom durch den Funktionsleiter 246 durch den zweiten Strombegrenzer 246 geleitet. Die in diesem Fall erzielbare Stromstärke bei einer vorgegebenen Betriebsspannung, die für den Fall einer vollständig intakten Strombegrenzungsvorrichtung ausgelegt ist, wird geringer ausfallen. Dies liegt an einem höheren Ohm'schen Widerstand, den der zweite Strombegrenzer 246 gegenüber einer Parallelschaltung des ersten und zweiten Strombegrenzers 244, 246 hat. Der zweite Strombegrenzer 246 ist so ausgelegt, dass die in diesem Fall erzielbare Stromstärke jedenfalls den zum zweckmäßigen Betrieb des medizinischen Geräts erforderlichen Minimalwert der elektrischen Stromstärke auf dem Funktionsleiter 226 ermöglicht.

Überbrückungskontakte 252 und 254 für den ersten Stromzweig und Überbrückungskontakte 256, 258 für den zweiten Stromzweig dienen dazu, bei einem eventuellen Ausfall eines oder sogar beider Strombegrenzer eine Überbrückungsmöglichkeit bereitzustellen. Auf diese Art und Weise kann bei einer defekten Strombegrenzungsvorrichtung 240 die Nutzung der Funktionsleitung 226 für therapeutische oder diagnostische Zwecke in einer Reparaturmaßnahme wieder ermöglicht werden. Bei einer einfachen Überbrückung mit Hilfe eines Kurzschlusses geht zwar die Strom begrenzende Wirkung verloren, jedoch erspart es dem Patienten die sonst erforderliche Operation zur Implantation einer neuen Elektrodenleitung. Solche Überbrückungsmaßnahmen können nämlich im implantierten Zustand, alternativ auch minimalinvasiv, erfolgen. Bei einer minimalinvasiven Reparatur werden die Überbrückungskontakte 252 und 254 oder 256 und 258 miteinander verbunden. Hierbei können sie beispielsweise mit Hilfe eines elektrischen Durchschlags oder durch Piezo-Verschweißen miteinander verbunden werden. Alternativ kann eine Reparatur erfolgen, indem über einem Mandrin ein Shunt zur Überbrückung des betreffenden Strombegrenzers eingebracht wird. Ein solcher Shunt kann beispielsweise durch ein gleitfähiges Wachs hergestellt werden. Dieses hat ebenfalls den Vorteil eines Schutzes vor Überhitzung im Falle von hohen induzierten Strömen, weil das Wachs bei Überhitzung schmilzt und die elektrische Verbindung zwischen den betreffenden Kontakten unterbrochen wird.

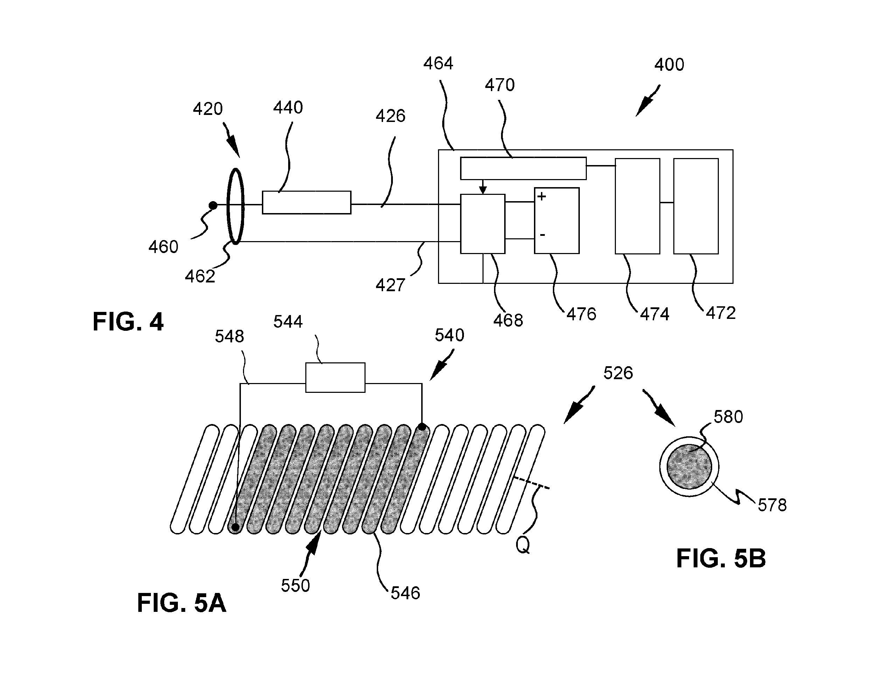

Fig. 4 ist eine vereinfachte Schaltskizze einer Elektrodenleitung nach einem weiteren Ausführungsbeispiel. Ein Herzschrittmacher 400, der ein aktives Implantat bildet, ist mit einer Elektrodenleitung 420 verbunden. Die Elektrodenleitung enthält Funktionsleiter 426 und 427. Der Funktionsleiter 426 verbindet ein Steuergerät 464 des Herzschrittmachers 400 mit einem Tip-Elektrodenpol 460. Eine zweite Funktionsleitung 427 verbindet das Steuergerät 464 mit einem Ring-Elektrodenpol 462. Die Funktionsleitung 426 ist mit einer Strombegrenzungsvorrichtung 440 verbunden. Es ist sinnvoll, die Strombegrenzungsvorrichtung nahe am distalen Tip-Elektrodenpol 460 anzuordnen, beispielsweise in einer Entfernung von weniger als 5 cm, gemessen entlang der Längserstreckung des Funktionsleiters 426.

Auf diese Weise kann die ungewünschte Erwärmung des Elektrodenpols unter Einfluss von hochfrequenten magnetischen Wechselfeldern besonders wirkungsvoll unterdrückt werden. Dies gilt unabhängig von der Form des Elektrodenpols, also beispielsweise unabhängig davon, ob es sich um einen Ring-Elektrodenpol oder einen Tip-Elektrodenpol handelt. Die Funktionsleitungen 426 und 427 sind mit einer Schalt-Einheit 468 verbunden. Die Schalt-Einheit wird von einer Steuer-Einheit 470 des Steuergeräts 464 gesteuert. Die Steuer-Einheit 470 ist ihrerseits eingangsseitig mit einer Messeinheit 472 und einer Auswerte-einheit 474 verbunden. Die Messeinheit 472 hat einen nicht näher dargestellten Messfühler, mit dem sie einen aktuellen Wert einer Messgröße erfasst, die geeignet ist, eine Wirksamkeit oder eine Unwirksamkeit der funktionellen Elektrodenpole 460 und 462 im jeweils aktuellen Betriebszustand zu ermitteln. Insbesondere eignet sich die Messgröße dazu, die Wirksamkeit oder Unwirksamkeit von Stimulationsimpulsen anzuzeigen, die an ein zu stimulierendes Gewebe abgegeben wurden.

RF-Impulse können beispielsweise durch die in eine RF-Antenne eingekoppelte Drehfelder oder in eine Kommunikationsspule eingekoppelte Gradienten/Wechselfelder oder durch einen Optokoppler, der induzierte Ströme in optische Signale umwandelt, detektiert werden.

Eine der Messeinheit 472 nachgeschaltete Auswerteeinheit ermittelt anhand des aktuellen Werts der Messgröße und vordefinierter Kriterien die Wirksamkeit oder Unwirksamkeit des aktuellen Betriebs der Funktions-Elektrodenpole 460 und 462, insbesondere also, ob Stimulationsimpulse an das umgebende Gewebe die erwünschte Wirkung haben. Die entsprechende Information wird an die Steuereinheit 470 ausgegeben.

Eine Energieversorgung 476 versorgt das Steuergerät 464 mit der im Betrieb benötigten Energie und erzeugt ebenso die für die Abgabe von Stimulationsimpulsen über die Schalt-einheit 468 benötigte Energie.

Nachfolgend wird näher auf eine mögliche Implementierung der Strombegrenzungsvorrichtung 440 eingegangen. Als Strombegrenzer zur Verwendung in der Strombegrenzungsvorrichtung eignet sich zunächst eine Diode, insbesondere ein PIN-Diode. Die Diode ist so verschaltet, dass die Abgabe von Stimulationsimpulsen im Normalbetrieb mit der dafür vorgesehenen Polarität nicht beeinflusst wird, insbesondere mit richtiger Polarität stimuliert wird. Nach Erfassung eines Ereignisses, das die Unwirksamkeit von Stimulationsimpulsen hervorruft, insbesondere also nach Detektion eines hochfrequenten magnetischen Wechselfeldes durch die Mess-Einheit 472 und die Auswerte-Einheit 444, sorgt die Steuereinheit 470 für das Anlegen einer Sperrspannung an die PIN-Diode der Strombegrenzungsvorrichtung. Hierfür wird die Schalteinheit 468 entsprechend gesteuert. Die PIN-Diode der Strombegrenzungsvorrichtung 440 sperrt und verhindert auf diese Weise einen Stromfluss mit einer Stromstärke durch den Funktionsleiter, die die Wirksamkeit von therapeutischen Stimulationsimpulsen beeinträchtigen würde. Um eine sperrende Vorspannung zu erzielen, wird der erforderliche Gegenpol über ein Gehäuse der Steuervorrichtung 464 oder einen anderen Elektrodenpol, wie beispielsweise den Ring-Elektrodenpol 462, an das im implantierten Zustand umgebende Körpergewebe angekoppelt.

Der zweite parallelgeschaltete Strumbegrenzer in der Strombegrenzungsvorrichtung 440 kann, muss aber keine PIN-Diode sein. Vorteilhaft ist anstelle einer weiteren PIN-Diode ein passiver Pfad, z.B. ein ohmscher Widerstand oder eine Vorrichtung oder ein Material das einen Widerstand realisiert. Der Widerstand dient als Sicherheitspfad über den eine Stimulation notfalls erfolgen kann wenn auch bei erhöhten Energiebedarf. Der Widerstand ist so zu wählen, dass er noch ausreichend MR Schutz bietet, bevorzugt 500ohm, bei mehreren solcher Schaltungen entlang der Elektrodenleitung in Summe bevorzugt von 1kohm oder mehr.

In einer alternativen Realisierungsmöglichkeit liegt die sperrende Vorspannung an der PIN-Diode der Strombegrenzungsvorrichtung 440 dauerhaft an und wird nur kurzzeitig abgeschaltet, wenn ein Stimulationsimpuls abgegeben werden soll. Dies kann durch Umpolung am Stromversorgungsanschluss selbsttätig erfolgen. Es sei angemerkt, dass bei dieser Realisierungsmöglichkeit der Leckstrom durch die PIN-Diode zuverlässig gering sein muss.

Fig. 5a und 5b zeigen eine schematische Darstellung einer Elektrodenleitung in einer seitlichen Ansicht und in einer Querschnittsansicht zur Erläuterung eines weiteren Ausführungsbeispiels. In diesem Ausführungsbeispiel wird eine Strombegrenzungsvorrichtung 540 in einem Funktionsleiter 526 mit Hilfe einer besonderen Ausbildung der Funktionsleitung selbst realisiert. Hierzu wird zunächst anhand der Fig. 5b eine Querschnittsansicht des Leiters in einer in Fig. 5a durch eine gepunktete Linie Q angedeuteten Querschnittsebene erläutert. Die Funktionsleitung wird durch den wendelförmig gewickelten Leiter realisiert, der einen elektrisch schlecht leitenden Kern 300 hat, welcher sich durch eine hohe mechanische Robustheit gegenüber den im Betrieb auftretenden mechanischen Belastungen auszeichnet. Der Kern ist in Fig. 5b durch eine graphische Textur gekennzeichnet. Der Kern ist umgeben von einer elektrisch gut leitfähigen Beschichtung 578. Ein durch den Leiter der Funktionsleitung 526 zu transportierender Strom wird aufgrund der unterschiedlichen elektrischen Leitfähigkeiten von Kern 580 und Beschichtung 578 überwiegend durch die Beschichtung 578 geführt. Zur Realisierung der Strombegrenzungsvorrichtung 540 ist in einem Längsabschnitt des Funktionsleiters 526 die gut leitfähige Beschichtung 578 entfernt. Der Funktionsleiter 526 bildet in diesem in Fig. 5a durch entsprechende Textur kenntlich gemachten Längsabschnitt einen relativ hohen Ohm'schen Widerstand. Dieser Längsabschnitt des Funktionsleiters bildet im vorliegenden Ausführungsbeispiel den zweiten Stromzweig 550, welcher dem zweiten Stromzweig 250 aus Fig. 2 entspricht. Durch eine Kontaktierung des Funktionsleiters 526 im Bereich des von der Beschichtung 578 befreiten Leiters wird der zweite Stromzweig 550 niederohmig überbrückt, wodurch ein erster Stromzweig 548 gebildet wird, der dem ersten Stromzweig 248 aus Fig. 2 entspricht. Dieser erste Stromzweig 548 enthält einen Strombegrenzer 544, der bei Stromstärken im Normalbetrieb, also im therapeutischen oder diagnostischen Betrieb, ohne störende Einflüsse, welche die Stromstärke im Funktionsleiter 526 erhöhen, einen niederohmigen Widerstand bildet.

Der Stromzweig 544 sollte, solange er im Bereich arbeitet in dem er den Strom noch nicht begrenzt, lediglich wenige mOhm aufweisen. Der hochomige Sicherheitspfad 550 soll im Bereich oberhalb von 500 Ohm liegen, bevorzugt über 1,0 Kiloohm liegen, um effektiv induzierte HF-Ströme zu dämpfen und dennoch über einen begrenzten Zeitraum die Funktionsfähigkeit des Implantats zu gewährleisten.

Anstelle der leitfähigen Beschichtung 578 kann auch eine andere Form der elektrisch gut leitfähigen Ummantelung verwendet werden. Eine gut leitfähige Beschichtung 578 hat den Vorteil, dass sie relativ leicht entfernt werden kann. Beispielsweise kann sie bei der Herstellung des Funktionsleiters 526 im gewünschten Längsabschnitt weggebrannt werden.

Das vorliegende Ausführungsbeispiel hat den Vorteil, dass relativ wenig Kontaktierungstechnik benötigt wird, um eine Parallelschaltung zweier Strombegrenzer zu realisieren. Aufgrund des hohen Ohm'schen Widerstands des vom Funktionsleiter selbst gebildeten zweiten Strombegrenzers 546 erfordert ein Betrieb nach Wegfall des ersten Stromzweiges 548 eine Anpassung der an die Funktionsleitung 526 abgegebenen Energie oder Leistung, um therapeutische Stimulationsimpulse mit einer Energie oder Leistung abgeben zu können, die bei Verwendung des niederohmigen ersten Stromzweiges erzielt werden kann. Hierzu wird auf das Ausführungsbeispiel der Fig. 4 verwiesen, insbesondere auf die Steuereinheit 470, welche im Betrieb die Schaltvorrichtung 468 ansteuern kann, eine entsprechende Anpassung der an die Funktionsleitung 426 oder 427 abgegebenen elektrischen Energie oder Leistung zu veranlassen.

Fig. 6a und 6b zeigen eine Schaltskizze und eine schematische Querschnittsansicht eines Strombegrenzers nach einem Ausführungsbeispiel. Das Schaltdiagramm der Fig. 6a zeigt einen Funktionsleiter 626 in einem Längsabschnitt, in dem eine Strombegrenzungsvorrichtung 640 vorgesehen ist. Die Strombegrenzungsvorrichtung enthält in ihrem ersten Stromzweig einen ersten Strombegrenzer 644, der aus zwei in Serie geschalteten Sperrschicht-Feldeffekttransistoren (englisch: Junction Field Effect Transistor, JFET) besteht. Die beiden JFETs 644.1 und 644.2 sind im vorliegenden Ausführungsbeispiel N-Kanal-JFETs. Sie sind an ihren Source- und Gate-Anschlüssen miteinander gekoppelt. Ihre Drain-Anschlüsse bilden die ein- und ausgangsseitigen Anschlüsse zum ersten Stromzweig 648. Der zweite Stromzweig 650 der Strombegrenzungsvorrichtung 640 enthält einen Strombegrenzer 646. Dieser kann beispielsweise, wie in Fig. 5a dargestellt, von einem hochohmigen Längsabschnitt des Funktionsleiters 626 gebildet sein. Die Schaltung der beiden JFETs 644.1 und 644.2 im ersten Strombegrenzer 644 kann mit Hilfe zweiter diskreter Transistorbauelemente realisiert werden. Alternativ können die beiden JFETs 644.1 und 644.2 in einem einzigen Halbleiterchip integriert werden. Dies wird weiter unten anhand von Fig. 6b näher erläutert.

Anstelle von JFETs ist auch die Verwendung von Metall-Halbleiter-Feldeffekttransistoren (MESFETs), von Feldeffekt-Transistoren hoher Elektronenbeweglichkeit, HEMTs oder von Metall-Oxid-Halbleiter-Feldeffekttransistoren (MOSFETs) möglich. Insbesondere MESFETs und HEMTs zeichnen sich durch ein besonders gut geeignetes Strombegrenzungsverhalten bei hohen Frequenzen aus, Frequenzbereichen also, in denen Magnetresonanz-Geräte arbeiten. Typischerweise liegen Frequenzen der von solchen Geräten erzeugten Wechselfelder im Bereich zwischen 1 und 150 MHz. Besonders gut geeignete HMETs sind pseudomorphe HMETs, die kurz auch als pHMET bezeichnet werden. Bei diesem Transistortyp bildet sich ein zweidimensionales Elektrodengas in einer pseudomorph verspannten dünnen Halbleiterschicht, welche besonders große Energieabstände der Leitungsbandunterkanten in dem Bandstrukturschema des Transistors ermöglicht. Dies erlaubt eine erhöhte Leistungsfähigkeit des Transistors.

Fig. 6b zeigt eine schematische Querschnittsansicht eines integrierten Halbleiterbauelements, das den Strombegrenzer 644 der Fig. 6a implementiert. In ein Halbleitersubstrat 680, das beispielsweise aus einem n-dotierten Halbleitermaterial, wie Silicium oder Galliumarsenid besteht, sind hoch p-dotierte Gebiete 682 eingebracht.

Das Gebiet 682.3 kann auf der Substratunterseite aufgebracht oder im Substrat vergraben werden. Wichtig ist hier, dass der Abstand von 682.1 & 2 zu 682.3 die Kanalbreite definiert, die neben der Dotierung den Abschnürpunkt (Pinch-off-Spannung Upo) bestimmt. Dies ist ein Parameter mit dem man neben dem Dotierverhältnis zwischen n und p+ die Strombegrenzung einstellen kann. Man kann diese Schicht auch vergraben. Dann muss der Wafer (Substrat) allerdings heruntergedünnt werden, so dass sich nicht unter der Schicht 682.3 ein weiterer Strompfad ausbilden kann. Alternativ kann auch in der Herstellung ein p-Substrat verwendet werden, wobei der Bereich 680 n zu Dotieren ist und die Bereiche 682.1&2 nachträglich wieder p+ zu dotieren sind..

N-dotierte Bereiche 684.1 und 684.2 sind beiderseits der hoch dotierten Gebiete 682.1 bis 682.3 oberflächennah in das Substrat eingebettet und mit Metallkontakten 686.1 und 686.2 versehen. Zur Steuerung des Kanals zwischen den hoch dotierten Gebieten 682.1 bis 682.3 sind die beiden hoch dotierten Gebiete 682.1 und 682.2 durch eine gemeinsame Metallschicht 686.3 kontaktiert.

Die Fig. 6B kann auch als eine geometrische Zusammenfassung der Schaltung aus Fig. 6A betrachtet werden. Damit ist das n+-dotierte Gebiet zwischen 682.1 und 682.2 das Drain des linken Transistors und Source des rechten Transistors. Zum Verständnis der Funktionsweise kann man in der Darstellung auch die Bereiche 684.1 und 684.2 und das Gebiet zwischen 682.1 und 682.2 weglassen. Die n+-Dotierung dient hier lediglich dem besseren Metall-Halbleiterübergang in dem n-dotierten Substrat und verhindert dass hier Shottkey-Dioden an den Übergängen entstehen.

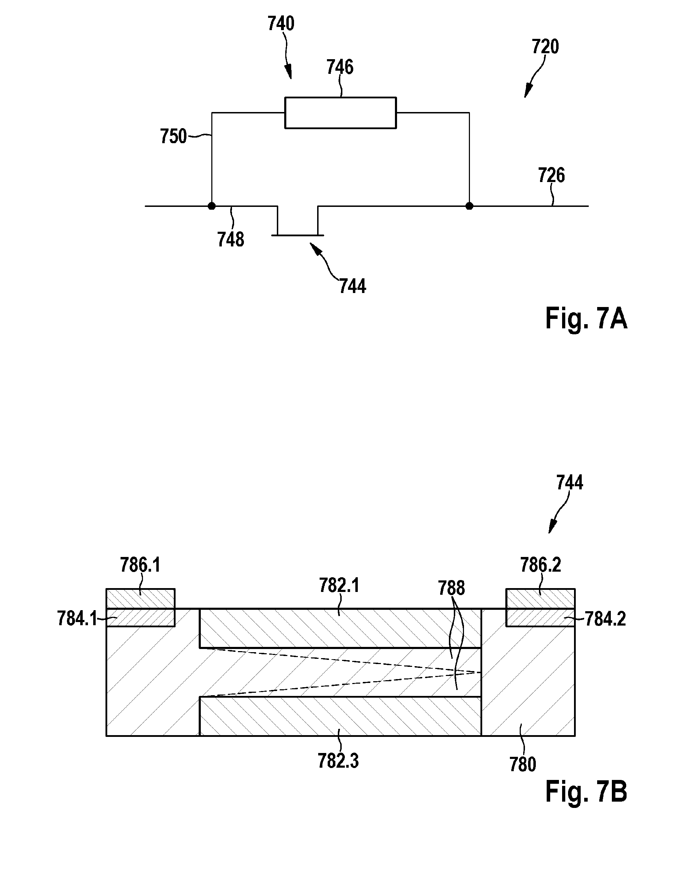

Fig. 7a und 7b zeigen eine Schaltskizze und eine schematische Querschnittsansicht eines alternativen Strombegrenzers. Die in Fig. 7 gezeigte Elektrodenleitung 720 hat in ihrem Funktionsleiter 726 eine Strombegrenzungsvorrichtung 740. Diese unterscheidet sich von der Strombegrenzungsvorrichtung 640 der Fig. 6a dadurch, dass der erste Strombegrenzer 744 im vorliegenden Ausführungsbeispiel aus nur einem einzigen JFET besteht. Der JFET 744 wird mit einem offenen Gate-Anschluss betrieben und weist einen symmetrischen Aufbau auf, der nachfolgend anhand von Fig. 7b beschrieben wird.

Die schematische Querschnittsansicht des als Strombegrenzer fungierenden JFETs 744 ähnelt grundsätzlich der Struktur des Strombegrenzers 644 der Fig. 6b. Die hoch dotierten Bereiche 682.1 und 682.2 sind vorliegend jedoch zu einem einzigen Gebiet 782.1 verschmolzen, die sich im Betrieb einstellende Verarmungszone 788 ist in Fig. 7b im Halbleitersubstrat 780 durch eine gestrichelte Linie dargestellt. Durch den symmetrischen Aufbau dieses Bauelements wirkt die Kanaleinschnürung 788 zwischen den Kontakten 786.1 und 786.2 in beide Stromflussrichtungen. Dies resultiert in einer Kennlinie des Strombegrenzers 744, wie sie in Fig. 3 durch die Bereiche 344.1 bis 344.3 dargestellt ist.

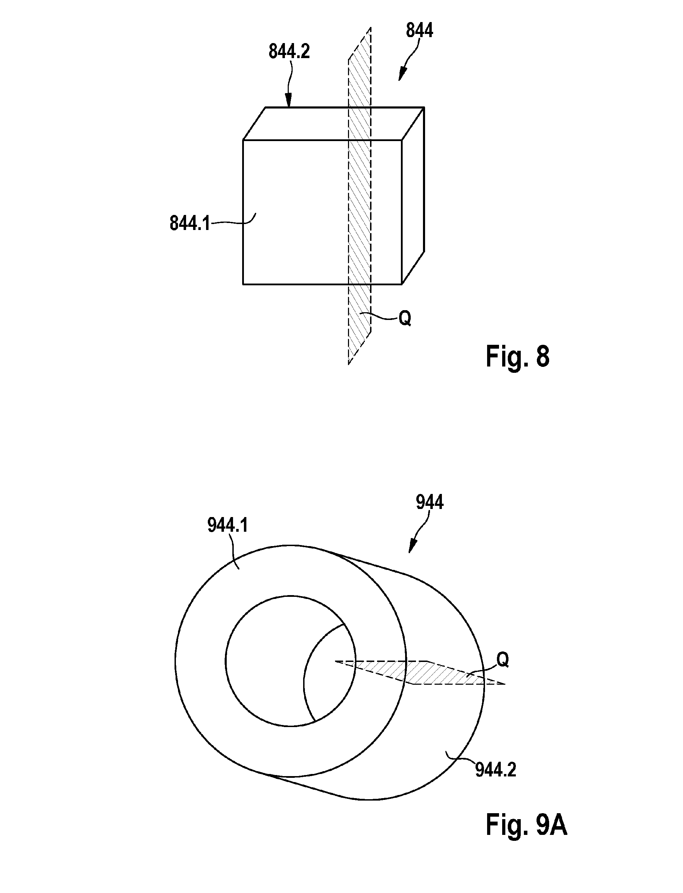

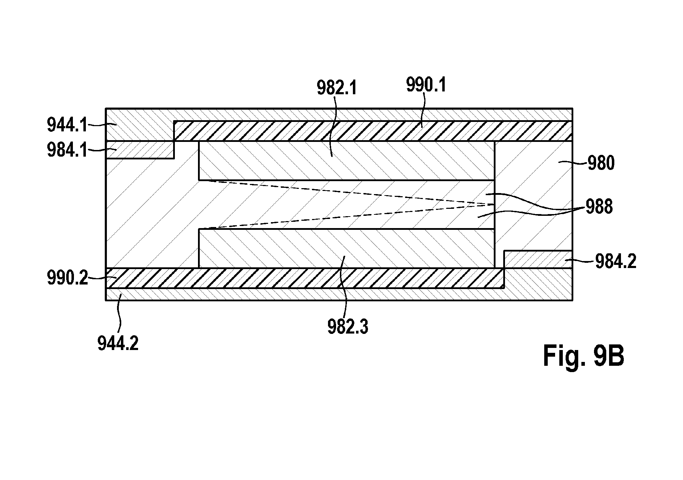

Fig. 8 zeigt in einer schematischen dreidimensionalen Ansicht einen Strombegrenzer 844. Der Strombegrenzer 844 ist als quaderförmiges Halbleiterbauelement ausgeführt, das an einer Frontfläche 844.1 und einer rückseitigen, in der Darstellung der Fig. 8 nicht sichtbaren und der Frontfläche 844.1 entgegengesetzten rückseitigen Fläche 844.2 mit einer Metallschicht zur Kontaktierung beschichtet ist. Der innere Aufbau eines solchen Bauelements wird weiter unten anhand von Fig. 9b erläutert.

Fig. 9a zeigt zunächst eine alternative Ausführungsform eines Strombegrenzers 944, der eine hohe zylindrische Form aufweist. Ein ringförmiger Bauelementbereich 944.1 umschließt eine kreisförmige Öffnung 944.2. Der Bauelementbereich ist an seiner ringförmigen Frontseite und seiner ringförmigen Rückseite mit einem Metall zur Kontaktierung beschichtet. Fig. 9b zeigt eine Querschnittsansicht, die den Aufbau der Strombegrenzer 844 und 944 in Querschnittsebenen Q darstellt. Der Aufbau entspricht im Wesentlichen der Struktur des Strombegrenzers 744 der Fig. 7b. Daher wird nachfolgend nur auf Unterschiede gegenüber dieser Struktur eingegangen. Zur besseren Einbettung des Bauelements in einen insbesondere wendelförmigen Funktionsleiter ist die frontseitige Metallfläche 944.1 mit dem eingangsseitigen n-dotierten Gebiet 984.1 verbunden. Eine Isolationsschicht 990.1 trennt die Metallkontaktierung vom hoch dotierten Gebiet 982.1. Einen entsprechenden Aufbau zeigt die rückseitige Metallfläche 944.2, die durch eine Isolationsschicht 990.2 vom hoch dotierten Gebiet 982.3 getrennt ist und außerhalb dieses Bereiches das n-dotierte Gebiet 984.2 kontaktiert.

Die Ausführung der Fig. 9 hat den Vorteil, dass diese Struktur des Strombegrenzers 944 in bekannte Strukturen von Elektrodenleitungen eingebettet werden kann. Beispielsweise ist mit einem wendelförmig geführten Funktionsleiter wie dem Funktionsleiter 526 der Fig. 5a eine besonders günstige Kontaktierung möglich. Mechanische Belastungen können bei Verwendung dieser Geometrie über die stabile Elektrodenleitung selbst aufgenommen werden, wodurch der gegenüber mechanischen Belastungen empfindliche Halbleiterchip entlastet wird.

Eine Ausführungsvariante ist, die mechanische Versteifung für die Tip-Wendel durchzuführen. So geht die mechanische Belastung beim Fixieren der Wendel ins Gewebe nicht auf das empfindliche Halbleiterbauelement, sondern kann durch eine mechanisch stabile Struktur durch die Durchführung übertragen werden.

## Patentansprüche

1. Temporär oder permanent implantierbares medizinisches Gerät mit mindestens einer langgestreckten elektrischen Funktionsleiter,
- der distal mit einem Elektrodenpol zur Abgabe von Therapiesignalen oder zur Erfassung von Diagnosesignalen verbunden ist,
- der in einem Längsabschnitt eine Strombegrenzungsvorrichtung aufweist, welche eine Parallelschaltung eines ersten und eines zweiten Strombegrenzers aufweist, von denen jeder ausgebildet ist, ein Überschreiten eines vorgegebenen Maximalwertes einer elektrischen Stromstärke im elektrischen Leiter zu verhindern,
- wobei der zweite Strombegrenzer zusätzlich ausgebildet ist, bei Anliegen einer elektrischen Betriebsspannung und gleichzeitigem Unterschreiten eines vorgegebenen, zum zweckgemäßen Betrieb des Gerätes erforderlichen Minimalwerts der elektrischen Stromstärke in einem ersten, nur den ersten der Strombegrenzer enthaltenden Stromzweig der Parallelschaltung, in einem zum ersten Stromzweig parallelen zweiten Stromzweig der Parallelschaltung, der nur den zweiten der Strombegrenzer enthält, eine elektrische Stromstärke zumindest des Minimalwerts zu gestatten.

2. Medizinisches Gerät nach Anspruch 1, bei dem eine elektrische Leitfähigkeit des ersten Strombegrenzers abhängig ist entweder von einer Frequenz eines Wechselfeldes, dem der erste Stromzweig ausgesetzt ist, oder von einer über dem ersten Stromzweig abfallenden elektrischen Spannung, oder von einem Betrag oder einer Richtung eines elektrischen oder magnetischen Feldes, dem der erste Stromzweig ausgesetzt ist, oder von einer Temperatur am ersten Stromzweig, oder von einer Kombination der genannten Größen.

3. Medizinisches Gerät nach Anspruch 1 oder 2, bei dem der erste Strombegrenzer mindestens eine Diode oder mindestens einen Transistor oder einen Schaltkreis mit mindestens einer Diode und mindestens einem Transistor enthält.

4. Medizinisches Gerät nach Anspruch 3, bei dem der Transistor ein Sperrschicht-Feldeffekttransistor, kurz JFET, ein Metall-Halbleiter-Feldeffekttransistor, kurz MESFET, ein Feldeffekt-Transistor mit hoher Elektronenbeweglichkeit, kurz HEMT, oder ein Metall-Oxid-Halbleiter-Feldeffekttransistor, kurz MOSFET ist.

5. Medizinisches Gerät nach Anspruch 3 oder 4, bei dem der erste Strombegrenzer von zwei in Reihe geschalteten Transistoren gebildet ist, deren Gate-Elektroden direkt miteinander verbunden sind.

6. Medizinisches Gerät nach einem der vorstehenden Ansprüche, bei dem der elektrische Leiter in seiner Längsrichtung Längsabschnitte von einander verschiedener, höherer oder geringerer elektrischer Leitfähigkeit aufweist, und bei dem der zweite Strombegrenzer von einem Längsabschnitt geringerer elektrischer Leitfähigkeit gebildet ist.

7. Medizinisches Gerät nach einem der vorstehenden Ansprüche, bei dem der erste Strombegrenzer als monolithisch integriertes Halbleiterbauelement in Form eines Hohlzylinders mit Kontaktflächen an seinen ringförmigen Längsenden ausgeführt ist, der den Funktionsleiter umgibt und bei Vorliegen eines Geräts nach Anspruch 6 den Längsabschnitt geringerer elektrischer Leitfähigkeit umgibt.

8. Medizinisches Gerät nach einem der vorstehenden Ansprüche, bei dem der Funktionsleiter an einem proximalen Ende seiner Längserstreckung eine Anschlussvorrichtung zum Anschluss an eine Steuervorrichtung des Geräts aufweist und an einem entgegengesetzten, distalen Ende seiner Längserstreckung mindestens einen zur Abgabe elektrischer Stimulationsimpulse an stimulierbares biologisches Gewebe geeigneten Elektrodenpol aufweist, wobei die Strombegrenzungsvorrichtung nahe dem distalen Ende des Leiters angeordnet ist.

9. Medizinisches Gerät nach einem der vorstehenden Ansprüche, bei dem der Funktionsleiter mit Überbrückungskontakten verbunden ist, die in Längsrichtung beiderseits der Strombegrenzungsvorrichtung angeordnet sind und die ausgebildet sind, durch Verbinden mit einer Überbrückungsvorrichtung direkt miteinander verbunden zu werden.

10. Medizinisches Gerät nach einem der vorstehenden Ansprüche, das zusätzlich eine Steuervorrichtung aufweist, die mit dem Funktionsleiter verbunden ist und die umfasst:
- eine Mess-Einheit, die ausgebildet ist, mit einem Messfühler einen Messwert einer von der elektrischen Stromstärke im Leiter verschiedenen Messgröße zu ermitteln, die geeignet ist, auf das Auftreten einer den Maximalwert überschreitenden elektrischen Stromstärke im Leiter hinzuweisen,
- eine Auswerte-Einheit, die ausgebildet ist, anhand des Messwerts das Vorliegen einer den Maximalwert überschreitenden elektrischen Stromstärke im Funktionsleiter zu erfassen und gegebenenfalls ein Steuersignal zu erzeugen,
- eine Schalt-Einheit, die mit der Steuervorrichtung und dem ersten Strombegrenzer verbunden ist und die ausgebildet ist, bei Anliegen des Steuersignals eine zum Sperren des durch den ersten Strombegrenzer führenden ersten Stromzweiges geeignete Sperrspannung zu erzeugen und an den Funktionsleiter anzulegen.

11. Medizinisches Gerät nach Anspruch 10, mit einer Therapie-Einheit, die mit der Auswerte-Einheit verbunden und ausgebildet ist,
- elektrische Stimulationsimpulse mit einem vordefinierten zeitlichen Verlauf ihrer Amplitude zu erzeugen, die einen vorgegebenen Sollwert der elektrischen Stromstärke im Funktionsleiter bewirken, wobei der Sollwert größer ist als der Minimalwert und kleiner als der Maximalwert,
- die Stimulationsimpulse über den Funktionsleiter nach extern abzugeben, und
- bei Anliegen des von der Auswerte-Einheit ausgegebenen Steuersignals den zeitlichen Verlauf der Amplitude der Stimulationsimpulse im Funktionsleiter zu ändern.

12. Medizinisches Gerät nach einem der Ansprüche 10 oder 11, bei dem
- die Mess-Einheit ausgebildet ist, einen aktuellen Wert einer Messgröße zu erfassen, die geeignet ist, eine Wirksamkeit oder eine Unwirksamkeit an das zu stimulierende Gewebe abgegebener Stimulationsimpulse anzuzeigen,
- die Auswerte-Einheit ausgebildet ist, anhand des aktuellen Werts und vordefinierter Kriterien die Wirksamkeit oder Unwirksamkeit der abgegebenen Stimulationsimpulse zu ermitteln, und
- die Therapie-Einheit ausgebildet ist, bei ermittelter Unwirksamkeit zeitlichen Verlauf der Amplitude der Stimulationsimpulse im Funktionsleiter zu ändern.

13. Medizinisches Gerät nach Anspruch 10 oder 11, bei dem der Messfühler am Funktionsleiter angeordnet und über den zweiten Signalpfad mit der Auswerte-Einheit verbunden ist.

14. Medizinisches Gerät nach einem der vorstehenden Ansprüche, bei dem das Gerät zusätzlich eine mit der Auswerte-Einheit verbundene Kommunikations-Einheit zur Kommunikation des Geräts mit einem externen Überwachungsgerät über einen Kommunikationskanal aufweist, wobei die Kommunikations-Einheit ausgebildet ist, bei Vorliegen des Steuersignals dem Überwachungsgerät auf dem Kommunikationskanal das Vorliegen einer den Maximalwert überschreitenden elektrischen Stromstärke im Funktionsleiter zu signalisieren.
